(19)

Europäisches
Patentamt

European
Patent Office

Office européen
des brevets

(11) **EP 4 032 986 A1**

(12) **EUROPEAN PATENT APPLICATION**
published in accordance with Art. 153(4) EPC

(43) Date of publication:
**27.07.2022 Bulletin 2022/30**

(21) Application number: **19946206.0**

(22) Date of filing: **09.12.2019**

(51) International Patent Classification (IPC):
***C12Q 1/6806*** (2018.01)

(86) International application number:
**PCT/CN2019/123892**

(87) International publication number:
**WO 2021/051665 (25.03.2021 Gazette 2021/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.09.2019 CN 201910896457**

(71) Applicant: **Shanghai Zenisight Ltd.
Shanghai 201210 (CN)**

(72) Inventors:
• **GUO, Zhiwei
Shanghai 201210 (CN)**
• **LI, Yinghui
Shanghai 201210 (CN)**
• **CHEN, Qian
Shanghai 201210 (CN)**
• **HU, Rongjun
Shanghai 201210 (CN)**

(74) Representative: **J A Kemp LLP
80 Turnmill Street
London EC1M 5QU (GB)**

(54) **ENRICHMENT METHOD AND SYSTEM FOR GENE TARGET REGION**

(57) The present invention provides an enrichment method for a gene target region, the method comprising: (1) amplifying, by means of a specific probe, fragmented DNA including a target region, and providing a captured extension product, wherein the specific probe comprises a sequence that is complementary to the target region of the fragmented DNA, and a 3'-end nucleotide and a 5'-end nucleotide of the probe are both modified; and (2) adding a ligase to the captured extension product provided in step (1), and providing a ligation product. The invention further provides an enrichment system for a gene target, the system being applicable to the enrichment method for a gene target region provided in the present invention.

FIG. 1

SEQUENCE LISTING

<110> SHANGHAI ZENISIGHT LTD.

<120> Enrichment method and system for gene target region

<130> N424021EP

<140> EP19946206.0
<141> 2019-12-09

<150> CN201910896457.8
<151> 2019-09-20

<160> 23

<170> PatentIn version 3.5

<210> 1
<211> 95
<212> DNA
<213> Artificial Sequence

<220>
<223> synthetic construct

<400> 1
gctgagtcgg agacacgcag ggatgagacg ccacaacagg ccttcaggct ctcgctgtgc      60

ccaatattgt ctttgtgttc ccggacatag tccag      95


<210> 2
<211> 95
<212> DNA
<213> Artificial Sequence

<220>
<223> synthetic construct

<400> 2
gctgagtcgg agacacgcag ggatgagacg ccacaacagg ccttcaggct ctcgctgtgc      60

ccaatattgt ctttgtgttc ccggacatag tccag      95


<210> 3
<211> 95
<212> DNA
<213> Artificial Sequence

<220>
<223> synthetic construct

<400> 3
gctgagtcgg agacacgcag ggatgagacg ccacaacagg ccttcaggct ctcgctgtgc      60

ccaatattgt ctttgtgttc ccggacatag tccag      95


<210> 4
<211> 95
<212> DNA

```
<213>  Artificial Sequence

<220>
<223>  synthetic construct

<400>  4
gctgagtcgg agacacgcag ggatgagacg ccacaacagg ccttcaggct ctcgctgtgc      60

ccaatattgt ctttgtgttc ccggacatag tccag                                 95


<210>  5
<211>  95
<212>  DNA
<213>  Artificial Sequence

<220>
<223>  synthetic construct

<400>  5
gctgagtcgg agacacgcag ggatgagacg ccacaacagg ccttcaggct ctcgctgtgc      60

ccaatattgt ctttgtgttc ccggacatag tccag                                 95


<210>  6
<211>  95
<212>  DNA
<213>  Artificial Sequence

<220>
<223>  synthetic construct

<400>  6
gctgagtcgg agacacgcag ggatgagacg ccacaacagg ccttcaggct ctcgctgtgc      60

ccaatattgt ctttgtgttc ccggacatag tccag                                 95


<210>  7
<211>  95
<212>  DNA
<213>  Artificial Sequence

<220>
<223>  synthetic construct

<400>  7
gctgagtcgg agacacgcag ggatgagacg ccacaacagg ccttcaggct ctcgctgtgc      60

ccaatattgt ctttgtgttc ccggacatag tccag                                 95


<210>  8
<211>  95
<212>  DNA
<213>  Artificial Sequence

<220>
<223>  synthetic construct

<400>  8
gctgagtcgg agacacgcag ggatgagacg ccacaacagg ccttcaggct ctcgctgtgc      60
```

```
ccaatattgt ctttgtgttc ccggacatag tccag                                95
```

<210>    9
<211>    95
<212>    DNA
<213>    Artificial Sequence

<220>
<223>    synthetic construct

<400>    9
```
gctgagtcgg agacacgcag ggatgagacg ccacaacagg ccttcaggct ctcgctgtgc    60

ccaatattgt ctttgtgttc ccggacatag tccag                                95
```

<210>    10
<211>    95
<212>    DNA
<213>    Artificial Sequence

<220>
<223>    synthetic construct

<400>    10
```
gctgagtcgg agacacgcag ggatgagacg ccacaacagg ccttcaggct ctcgctgtgc    60

ccaatattgt ctttgtgttc ccggacatag tccag                                95
```

<210>    11
<211>    96
<212>    DNA
<213>    Artificial Sequence

<220>
<223>    synthetic construct

<400>    11
```
gctgagtcgg agacacgcag ggatgagacg ccacaacagg ccttcaggct ctcgctgtgc    60

ccaatattgt ctttgtgttc ccggacatag tccagt                               96
```

<210>    12
<211>    20
<212>    DNA
<213>    Artificial Sequence

<220>
<223>    synthetic construct

<400>    12
```
ccttcaggct ctcgctgtgc                                                 20
```

<210>    13
<211>    20
<212>    DNA
<213>    Artificial Sequence

```
<220>
<223>   synthetic construct

<400>   13
cctgttgtgg cgtctcatcc                                                    20


<210>   14
<211>   22
<212>   DNA
<213>   Artificial Sequence

<220>
<223>   synthetic construct

<400>   14
tcccggacat agtccaggag gc                                                 22


<210>   15
<211>   18
<212>   DNA
<213>   Artificial Sequence

<220>
<223>   synthetic construct

<400>   15
cgtgtgccgc ctgctggg                                                      18


<210>   16
<211>   17
<212>   DNA
<213>   Artificial Sequence

<220>
<223>   synthetic construct

<400>   16
cacggtggag gtgaggc                                                       17


<210>   17
<211>   185
<212>   DNA
<213>   Artificial Sequence

<220>
<223>   synthetic construct

<400>   17
gatccttcag gctctcgctg tgcccaatat tgtctttgtg ttcccggaca tagtccagga       60

ggcagccgaa gggcatgagc tgcgtgatga gctgcacggt ggaggtgagg cagatgccca      120

gcaggcggca cacgtggggg ttgctgagtc ggagacacgc agggatgaga cgccacaaca      180

ggatc                                                                   185


<210>   18
<211>   90
```

```
<212>  DNA
<213>  Artificial Sequence

<220>
<223>  synthetic construct

<400>  18
gctgagtcgg agacacgcag ggatgagacg ccacaacagg ccttcaggct ctcgctgtgc      60

gctgccgaag aattgcagtt tgcttcccgc      90


<210>  19
<211>  11
<212>  DNA
<213>  Artificial Sequence

<220>
<223>  synthetic construct

<400>  19
gcgtgatgag t      11


<210>  20
<211>  16
<212>  DNA
<213>  Artificial Sequence

<220>
<223>  synthetic construct

<400>  20
cgcttcggta ctctgc      16


<210>  21
<211>  19
<212>  DNA
<213>  Artificial Sequence

<220>
<223>  synthetic construct

<400>  21
gtcatccctg tacaacctg      19


<210>  22
<211>  19
<212>  DNA
<213>  Artificial Sequence

<220>
<223>  synthetic construct

<400>  22
gcgaagtgca actggacaa      19


<210>  23
<211>  19
<212>  DNA
```

```
<213>  Artificial Sequence

<220>
<223>  synthetic construct

<400>  23
tgcgtgtctc cgactcagc                                                    19
```

**Description**

**TECHNICAL FIELD**

The present invention relates to the field of biotechnology, more particularly, to an enrichment method and system for a gene target region.

**BACKGROUND OF THE INVENTION**

Gene sequencing technology has undergone nearly half a century since it appears in the 1970s. The emergence of PCR technology in 1985 has promoted the development of the entire molecular biology. Next-generation sequencing technology (NGS) has the advantages of accuracy, sensitivity, and high throughput. As the cost of sequencing continues to decrease, the application range of NGS continues to expand, but its applications are also limited by diverse requirement and time-consuming laborious library construction. In the process of constructing clinical samples such as plasma samples, the forms of DNA are usually short-fragmented, damaged, single-stranded or partially double-stranded, for DNA of such existing forms, especially fragments of less than 200 bp, current PCR technology cannot achieve good capture and enrichment.

For the enrichment of tiny DNA fragments, the prior arts still mainly use a conventional PCR library-constructing method, or first adding a linker and then amplifying, such as the hybrid-capture method. However, for the former, due to the requirement for a double-ended primer, the length of the fragment suitable for amplification is greatly limited, and the preference in amplification results in high heterogeneity of the product and the error accumulated by the exponential amplification leads to inaccurate subsequent sequencing results. For the latter, although the requirement for the length of the fragment to be enriched is not as strict as PCR, the ligation reaction needs to be performed first, and the efficiency of the ligation reaction usually is only 20% -50%, resulting in low capture efficiency as well as the problem of being easy to lose rare molecules due to difficulty in ligation. In order to solve the problem of NGS library construction, technologies such as molecular inversion probes, multiple PCR and the like are recently developed. Compared with hybrid-capture technology, the molecular inversion probes have better specificity, but its pocket-like probe is complex in design and is not suitable for enrichment of tiny DNA fragments. Multiple PCR technology is suitable for large-scale samples and is most widely used. But neither of them is suitable for enrichment for tiny DNA fragments at low starting concentration, since either the requirements to primer design are extremely high and the homogeneity of the amplicon is poor, or the homogeneity of the amplified product is good but the requirement to the concentration of the starting sample is very high. These prior arts usually require double-ended primers to construct libraries, so, in order to remove linker dimer contamination, a purification step have to be introduced, which results in the loss of information in small fragments of double-stranded DNA, damaged double-stranded DNA, and single-stranded DNA molecules. However, the genomic regions that are active in some transcriptions are just the DNA in these forms. To sum up, the prior arts can't satisfy the requirement for enrichment of fragmented, especially tiny DNA of 200bp or less. Therefore, the main technical purpose of the present invention is to provide a method for efficiently enriching the target regions in fragmented DNA, breaking the bottleneck of enrichment efficiency limited by low ligation efficiency, inhibiting the production of unintended ligation products, and maximally increasing the capture efficiency of rare molecules while maintaining the uniformity of the product. In the application No. 2019100024085, the applicant has established a technical solution of linearly amplifying the target region with a specific probe firstly and then ligating linkers to achieve enrichment. The main application direction is the nucleic acid detection based on second-generation sequencing. The present invention will solve the technical problem of targeted enrichment of fragmented DNA with another idea.

**SUMMARY OF THE INVENTION**

In view of the shortcomings of the prior art described above, the object of the present invention is to provide an enrichment method for a gene target region so as to solve the problems in the prior art.

To achieve the above objects and other related objectives, firstly, the present invention provides an enrichment method for a gene target region comprising:

(1) amplifying fragmented DNA comprising a target region by means of specific probe to provide a captured-extension product; wherein the specific probe comprises both a sequence complementary to the target region of the fragmented DNA and a sequence not complementary to the target region of the fragmented DNA, and a 3'-end nucleotide and a 5'-end nucleotide of the specific probe are both modified;

(2) adding a ligase to the captured extension product provided in step (1) to provide a ligated product, which includes a circular ligated product and linear ligated product.

Another object of the present invention is to provide a system for enriching fragmented DNA target regions, comprising specific probes and ligases applicable to the gene target region enrichment method provided by the present invention.

**BRIEF DESCRIPTION OF THE DRAWINGS**

FIG. 1 is a schematic flowchart of an enrichment method for a target region in an embodiment of the present invention.
FIG. 2 is a schematic diagram of a library molecule constructed according to an embodiment of the present invention.

**DETAILED DESCRIPTION OF THE EMBODIMENTS**

The inventors of the present invention provided an enrichment method for gene target region after a lot of exploratory research. The enrichment method for gene target region is easy to operate and has reliable results, especially a good enrichment effect for short-length nucleic acid. Based on these, the present invention has been completed.
The first aspect of the present invention is to provide an enrichment method for a gene target region, comprising:

(1) Amplifying the fragmented DNA containing a target region by specific probe to provide a captured-extension product, and the specific probe includes both sequences complementary to the target region of the fragmentation DNA and sequences not complementary to the target region of the fragmentation DNA. Both the 3'-end and 5'-end nucleotides of the specific probe are modified to prevent the ligation reaction at the 3'-end of the specific probe, mainly self-ligation reaction with its own 5'-end, before the probe binds to the template.
(2) Adding ligase to the captured extension product provided in step (1) to provide a ligated product. The ligated product in this step includes two parts, one is a circular ligated product formed by intramolecular ligation of the captured extension product, and the other is a linear ligated product formed by intermolecular ligation of the captured extension product and the specific probe molecule. Both the circular ligated product and the linear ligated product contain the target region that needs to be amplified, and are suitable for the library construction of target molecules.

In the enrichment method for a gene target region provided by the present invention, the fragmented DNA containing the target region is amplified by a specific probe, and the fragmented DNA containing the target region may be one or multiple. Generally speaking, there is a one-to-one correspondence between specific probe and fragmented DNA containing target region, that is, the number of specific probes in the reaction system may be one or more. Extension described in this step refers to a complete linear pre-amplification step for the sample, including a cycle of single or multiple rounds of denaturation, annealing, and extension steps. In a preferred embodiment, multiple cycles, such as 2-100, 2-10, 10-20, 20-30, 30-40, 40-60, 60-80, 80-100 cycles, were performed in this step, effectively increase the number of molecules that contain the target region.
In the enrichment method for a gene target region, the fragmented DNA can be double-stranded DNA, single-stranded DNA, cDNA, etc., and cDNA can usually be obtained by reverse transcription of RNA. For double-stranded DNA, specific probe may also include sequence complementary to the target region of one strand of the fragmented DNA. So, the enrichment method of the present invention is also applicable to fragmented RNA. Those skilled in the art can reverse-transcribe the RNA into cDNA and then perform subsequent operations by the enrichment method provided in the present invention. Fragmented DNA can be 25-200bps, 25-40bps, 40-60bps, 60-80bps, 80-100bps, 100-120bps, 120-140bps, 140-160bps, 160-180bps, or 180-200bps in length.
In the enrichment method for a gene target region, the amplification system in step (1) may include specific probe, DNA polymerase, and dNTP. The reaction of amplifying the fragmented DNA comprising the target region by specific probe is usually carried out in the presence of DNA polymerase. After the probe with 3'-end blocking-modified bind to the template under the action of high-fidelity polymerase, the blocked group is excised and the probe is activated, so that the target sequence can be effectively extended. The DNA polymerase may have 3'-5' exonuclease activity, so that the substituent group at the 3'-end of the probe after binding to the template can be excised and the probe can be extended along the template, preferably a high-fidelity DNA polymerase, so as to further improve the amplification efficiency and purity of the product. The DNA polymerase can also be a common DNA polymerase, that is, only having polymerase activity, but no 3'-5' exonuclease activity. In this case, the amplification system of the step (1) also includes an active substance, the active substance can be used to excise the 3'-end modification group of the specific probe after bound to the target region, so it can be combined with DNA polymerase (for example, ordinary DNA polymerase) in the captured-extension system to improve the efficiency of the linear amplification system. The above active substance is preferably a nuclease. The amplification reaction of the fragmented DNA containing the target region by specific probe is usually performed under the presence of dNTP, which may generally be a dNTP coupled to a labeling molecule, and the coupled labeling molecule may include, but not limited to, biotin, and the like, and the dTNP may include, but not limited to, dCTP, dATP, and the like. The dNTP can also be coupled with a labeling molecule, which can be biotin, and the like, and the labeling molecule may usually be used for the purification of the captured extension product.

In the enrichment method for the gene target region, the specific probe comprises a sequence complementary to the target region of the fragmented DNA, so that the specific amplification of the target region of the fragmented DNA can be achieved. Those skilled in the art can select a suitable target region of the fragmented DNA and design a suitable complementary sequence according to the target region of the fragmented DNA, and the complementary sequence is usually close to the 3'-end of the probe. The specific probe usually also includes a sequence that is not complementary to the fragmented DNA, so that an enzyme-digestion site and a universal sequence can be introduced, and the non-complementary sequence is usually close to the 5'-end of the probe. The specific probe can be a specific probe whose 3'-end nucleotide is modified to prevent the 3'-end of the specific probe from ligating with other groups, so as to prevent free probes from self-ligating or ligating with other non-target molecules. Those skilled in the art can select a suitable modification group to achieve modification of the 3'-end of the specific probe, for example, the modified group can replace the natural group on the nucleotide at the 3'-end of the specific probe (for example, hydroxy, methoxy, etc.) to prevent the 3'-end of the specific probe from ligating, and the modification group can usually be a blocking group. After the probe is combined with the target region on the template through the complementary sequence, the modification group at the 3'-end can be excised by the enzyme to make the probe be activated, so that the target sequence can be effectively extended. The 3'-end modification group of the specific probe can include, but not limited to, a hydrogen atom, a C3 Spacer group, a C6 Spacer group, a phosphate group ($PO_4$), an amino group ($NH_2$), and the like. Different substituent groups have obvious differences in capture effect of the probe. In a preferred embodiment of the present invention, the effect obtained by substituting the 3'-end hydroxyl group of the probe with C3 Spacer is the best, and it has obvious advantage compared with other substituent groups. The specific probe also includes a universal sequence, which can usually be recognized by the sequencing system, so that the ligated product provided subsequently can be sequenced by the sequencing system, for example, for the Ion Torrent sequencing system, the universal sequence can be the corresponding PI sequence and the reverse complementary sequence of A sequence. Use of the sequence that can be recognized by the sequencing system enables the library constructed by the present invention to be sequenced through a high-throughput sequencing platform, so as to provide information required for various subsequent studies and clinical applications. In a preferred embodiment of the present invention, the base in the tail region at the 3'-end of the specific probe may contain a mismatch, and the mismatch may be the last base at the 3'-end of the probe, or it may be the base close to the 3'-end. The mismatch may be one base or multiple bases. The mismatch not only have no effect on the specificity and binding efficiency of the probe, but also is more conducive to improve the cutting efficiency and fidelity of the high-fidelity DNA polymerase.

In the enrichment method for the gene target region, in step (1), purification of the captured extension product may also be included. Those skilled in the art can choose a suitable method to purify the captured extension product, for example, the purification method of the captured extension product can include silica gel column purification, heat treatment, magnetic bead purification and the like. In an embodiment of the present invention, the captured extension product can be purified against the dNTP coupled labeled molecule, and avidin or streptavidin-coated magnetic beads may be used in the purification process.

The enrichment method for a gene target region provided by the present invention may further include: adding a ligase to the captured extension product provided in step (1) to provide a ligated product. The ligase is a single-stranded ligase, preferably T4 RNA ligase or thermostable RNA ligase and the like. The ligated product can be a circular ligated product formed by intramolecular ligation of the captured extension product, or a linear ligated product formed by intermolecular ligation between the captured extension product and a specific probe molecule.

In the enrichment method for a gene target region, the specific probe may be modified at 5'- end nucleotide and may be a single-stranded structure at the reaction temperature of step (2), so that the ligated product can be obtained under the catalysis of the single-stranded ligase by means of the 3'-end hydroxyl group of the captured extension product forming a covalent bond with the modified group at the 5'-end of itself or the specific probe. In an embodiment of the present invention, the 5'- end nucleotide of the specific probe (for example, the 5-position hydroxyl group of the 5'-end nucleotide) is substituted by a phosphate group, thereby forming a phosphorylated modification. Under the catalysis of single-stranded ligase, the 3'-end hydroxyl group of the captured extension product forms a covalent bond with the phosphorylated 5'-end to obtain a ligation product. At this time, the specific probe has two states, one is a specific probe with a phosphorylated 5'-end that binds to the template to form a captured extension product, and the other is a free specific probe. Therefore, the corresponding ligation reaction can also be in two cases, one is that the 3'-end hydroxyl group of the captured extension product is ligated to its own 5'-end phosphate group, with the end-to-end ligation in the molecule forming a ring; the other is that the 3'-end hydroxyl group of the captured extension product is ligated with the 5'-end phosphate group of the free probe, and the ligated product is still a linear product. In another embodiment of the present invention, the 5'-end nucleotide of the specific probe (e.g, the 5-position hydroxyl group of the 5'-end nucleotide) is substituted with an adenosine group, thereby forming adenosine modification. Under the catalysis of 5'App DNA/RNA thermostable ligase, the captured extension product can also be circularly ligated intramolecularly, or the adenosine group at the 5'-end of the free probe can be ligated to the 3'-end of the captured extension product. In another embodiment of the present invention, the 5'-end nucleotide of the specific probe (for example, the 5-position hydroxyl group of the 5'-end nucleotide) is replaced by a phosphate group, thereby forming a phosphorylated modification. Under the catalysis of thermostable RNA ligase, the 5'-end of the specific probe can be ligated with the 3'-end of the captured extension product, and two kinds of ligated products, intramolecular circular ligated products and intermolecular linear ligated products, are generated. The specific probe can also be a partial double-stranded structure with a sticky end in the 5'-end region, and the sticky end in the 5'-end region has a single-stranded property, so that the 5'-end can be modified by the above-mentioned method in order to be ligated to the captured extension product under the catalysis of a suitable single-stranded ligase. In the enrichment method for the gene target region, the step (2) may further include purification of the ligated product. Those skilled in the art can choose a suitable method to purify the ligated product, for example, the purification method for the ligated product may include but not limited to silica gel column purification, heat treatment, magnetic bead purification and the like.

The enrichment method for a gene target region provided by the present invention may further include: (3) in the sequence of the specific probe which is not complementary to the target region, usually the sequence near the 5'-end is provided with enzyme-digestion site, and after the step (2), an endonuclease is added for excising the circular ligated product in the ligated product formed in the step (2) at the enzyme-digestion site, so that the circular ligated product is converted to be linear ligated product, which is beneficial to the subsequent PCR amplification and PCR detection reaction. In another embodiment of the present invention, the enzyme-digestion site is uracil U that replaced the original thymine T, and the endonuclease is USER enzyme or SSDNA endonuclease. In another embodiment of the present invention, there is an enzyme-digestion site U near the 5'-end of the specific probe. After the captured extension products are ligated end to end to form a circular product, the USER enzyme or SSDNA endonuclease cut the circular product at the enzyme-digestion site U to make it to be a liner ligated product, and exponential amplification can be achieved by means of making PCR amplification primers combining to the linear ligated product after excised by enzyme.

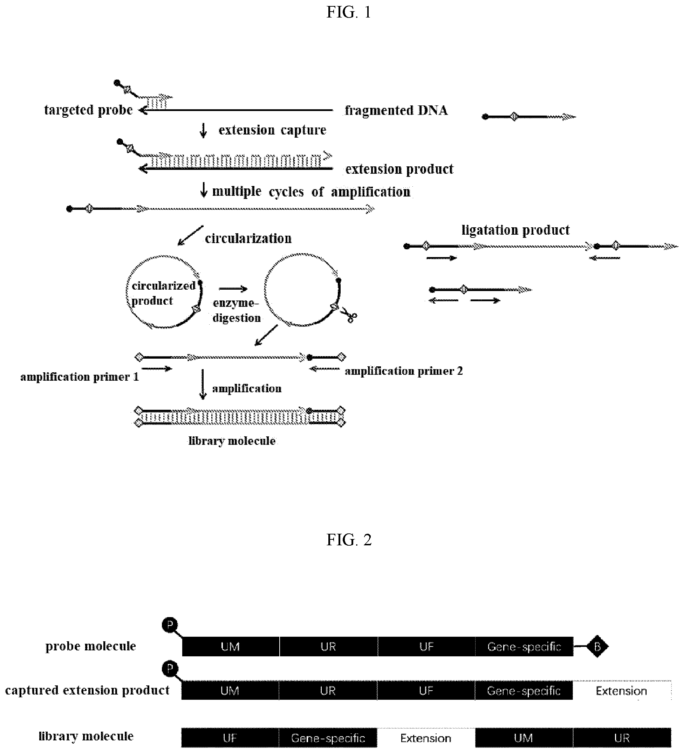

The enrichment method for a gene target region provided by the present invention may further include: (4) PCR amplifying the ligated product provided in step (2). The PCR amplification primer has a sequence complementary to the specific probe, and the complementary sequence is not complementary to the sequence of the target region. The enrichment method for a gene target region provided in the present invention may further include: (5) after the step (3), PCR amplifying the ligated products provided in the steps (2) and (3), the PCR amplification primer has a sequence complementary to the sequence on both sides of the enzyme-digestion site, preferably, the sequence on both sides of the enzyme-digestion site is a universal sequence for sequencing. Usually, ligated product can be PCR amplified by DNA polymerase and PCR amplification primer, the product containing the DNA of the target region can be further enriched by amplifying the ligated product. Those skilled in the art can choose a suitable method and system to amplify the ligated product provided in step (2) or step (3). For example, in an embodiment of the present invention, the PCR amplification primer has the complementary and reverse complementary sequences to the sequence near the 5'-end of the specific probe not bound to the template, it can amplify the circular ligated product together with the linear ligated product provided in step (2). In another embodiment of the present invention, there is an enzyme-digestion site U near the 5'-end of the specific probe, and the primers before and after PCR amplification are complementary and inverse complementary to the sequences on both sides of the enzyme-digestion site U of the specific probe, respectively. When USER enzyme or ssDNA endonuclease excised the circular ligated product into linear ligated product at the enzyme-digestion site U, the PCR amplification primer can combine the two ends of the excised linear ligated product through the complementary sequence to achieve an exponential amplification, as shown in Figure 1. In the enrichment method for a gene target region, the step (4) or (5) may further include purifying the amplified product. Those skilled in the art can choose a suitable method, including but not limited to silica gel column purification, heat treatment, magnetic bead purification and the like, to purify the amplified product.

The enrichment method for a gene target region provided by the present invention may further include: (6) detecting the ligated product provided in step (4) or (5) using detection primer 1, detection primer 2 and probe 3, and providing quickly the detection results of the target region using PCR detection method instead of second-generation sequencing. At least one of the above-mentioned detection primer 1, detection primer 2 and probe 3 contains gene-specific sequences, that is to say, for different gene target regions, the combination of the specific sequences of the three primers/probes can be monospecific, bispecific or tri-specific. In an embodiment of the present invention, the detection primer 1 includes a gene-specific sequence, the detection primer 2 and the probe 3 include universal sequence; or the detection primer 1 and the detection primer 2 include a gene-specific sequence; or the detection primer 1 and the probe 3 contains a gene-specific sequence; or all of detection primer 1, detection primer 2, and probe 3 contain a gene-specific sequence. The probe 3 may further include a labeling molecule, such as a fluorescent molecule, and the sequence of probe 3 is not complementary to that of detection primer 1 or 2.

The enrichment method for a gene target region provided by the present invention can be used for nucleic acid detection. Methods for further detection by amplified ligated products are known to those skilled in the art. The enrichment method for a gene target region of the present invention can be applied to PCR-based gene sequencing detection. For example, the target region includes, the site of sequence variation, more specifically, the single-base mutation site region, the base deletion site region, base insertion site region, fusion mutation site region, epigenetic variation or gene specific sequence, etc. In an embodiment of the present invention, the enrichment method for a gene target region provided by the present invention achieved ideal results when applied to the detection of EGFR SNP-Q787 site mutation and male-specific SRY gene.

The second aspect of the present invention provided a system for enriching target regions of fragmented DNA, including specific probes and ligases suitable for the enrichment method for a gene target region provided in the first aspect of the present invention. Wherein, the ligase can be thermostable RNA ligase, T4 RNA ligase, or 5'App DNA/RNA thermostable ligase and the like. The structure of the specific probe has been described in detail in the first aspect of the present invention, and are omitted here.

The system provided by the present invention may further included one or more of the following components: dNTP coupled with labeling molecule, DNA polymerase, nuclease, endonuclease, etc., in which, dTNP may be dCTP or dATP. The coupled labeling molecule may be biotin. The DNA polymerase may be a DNA polymerase with 3'-5' exonuclease activity, preferably a high-fidelity DNA polymerase. The nuclease has 3'-5' exonuclease activity. The endonuclease may be a USER enzyme or a ssDNA endonuclease.

The system provided by the present invention can further include PCR amplification primers, the sequences of which are usually matched with the universal sequences of the specific probes and the sequences on both sides of the circular enzyme-digestion site, in detail, the sequences are complementary to the sequence on both sides of the circular enzyme-digestion site and are at least partially complementary to the universal sequence of the specific probe.

The system provided by the present invention may also include detection primer 1, detection primer 2 and probe 3 used for PCR detection, and at least one of them contains a gene-specific sequence, in detail, monospecific, bispecific, tri-specific primer/probe or their combinations. In a preferred embodiment, only the detection primer 1 comprises a gene-specific sequence. It is applied to PCR detection instead of second-generation sequencing.

In a preferred embodiment of the present invention, after the library is constructed by the method or system of the present invention, the library molecules sequentially comprise the following sequences in structure: 5'-end sequencing universal sequence, gene-specific probe sequence, enriched target region sequence and the 3'-end sequencing universal sequence, as shown in Fig. 2. The enriched target region contains the sequence information of the DNA sample before enrichment, wherein the position of the 5'-end on the genome is fixed and is determined by the specific probe; while the position of the 3'-end is not fixed and is determined by the initial state of DNA fragment at the beginning of library construction. Therefore, in the data analysis after enrichment, the position of the 3'-end of the sequence on the genome can also act as a molecular tag. The beneficial effects of the present invention are as follows:

Firstly, before the ligation, the target regions of all fragmented DNA samples are pre-amplified by means of captured extension, to avoid loss or missed detection of original target molecules, especially small fragments and rare molecules, caused by insufficient ligation efficiency of ligase during the ligation stage. The extension reaction in the pre-amplification stage is linear amplification and has no preference of PCR amplification, so, it will not accumulate errors introduced by PCR amplification. Compared with the conventional PCR library constructing technology, the product is more uniform.

Secondly, in the pre-amplification stage, only a single-stranded probe with a length of about 30 bp is necessary to be designed for each target gene, which can avoid the difficulty of designing double-ended primers for short fragments such as cfDNA. Thus, not only the success rate of library construction but also the convenience of library construction is improved. Blocking the 3'-end of the probe can block the non-intended ligation except ligation of the probe and the template, effectively reducing the background noise caused by the free probe, supplemented by the dNTP coupled to the labeling molecule and its purification system, the purity of the target product can be further improved and the DNA sample molecules can reach theoretical maximum conversion rate.

Thirdly, after the 5'-end of the specific probe is modified, it can be well ligated to the 3'-end of the extension product under the catalysis of single-stranded ligase and will not be ligated to the 3'- end of the block-modified free probe, self-ligation or mis-ligation of the free probe are avoided and the proportion of the target ligation product in the final product is increased.

Finally, compared with the enrichment method disclosed in the application No. 2019100024085, the enrichment method of the present invention omits the linker DNA, so, it is more convenient in the preparation of raw materials, and the chance of generating undesired products due to interference between different components is lower, and the cost is further reduced.

To sum up, the enrichment method and system of the present invention is simple in operation and the results is reliable. When used for DNA with a fragment length of less than 200 bp, the enrichment method and system of the present invention can minimize the loss of original molecules, especially rare molecules, and can enrich target molecules most efficiently. In addition, the fidelity, specificity and sensitivity are high, and it can detect rare mutant molecules with a mutation rate as low as 0.01%.

The embodiments of the present invention are described below through examples, and those skilled in the art can easily understand other advantages and effects of the present invention from the contents disclosed in this description. The present invention can also be implemented or applied through other different embodiments, and various details in this description can also be modified or changed based on different viewpoints and applications without departing from the spirit of the present invention.

**Example 1**

**The oligonucleotide used in Example 1 are shown in table 1:**

Table 1

| Name | SEQ ID NO. | 5'-end modification | 3'-end modification | Sequence (5'→3') |
|------|-----------|--------------------|--------------------|------------------|
| Probe1 | 1 | 5'OH | 3'OH | GCTGAGTCGGAGACACGCAG GGATGAGACGCCACAACA**GG CC**TTCAGGCTCTCGCTGTGC CCAATATTGTCTTTGTGTTC CCGGACATAGTCCAG |
| Probe2 | 2 | 5'PO4 | 3'OH | GCTGAGTCGGAGACACGCAG GGATGAGACGCCACAACA**GG CC**TTCAGGCTCTCGCTGTGC CCAATATTGTCTTTGTGTTC CCGGACATAGTCCAG |
| Probe3 | 3 | 5'PO4 | 3'C6 Spacer | GCTGAGTCGGAGACACGCAG GGATGAGACGCCACAACA**GG CC**TTCAGGCTCTCGCTGTGC CCAATATTGTCTTTGTGTTC CCGGACATAGTCCAG |
| Probe4 | 4 | 5'PO4 | 3'C3 Spacer | GCTGAGTCGGAGACACGCAG GGATGAGACGCCACAACA**GG CC**TTCAGGCTCTCGCTGTGC CCAATATTGTCTTTGTGTTC CCGGACATAGTCCAG |
| Probe5 | 5 | 5'PO4 | 3'NH$_2$ | GCTGAGTCGGAGACACGCAG GGATGAGACGCCACAACA**GG CC**TTCAGGCTCTCGCTGTGC CCAATATTGTCTTTGTGTTC CCGGACATAGTCCAG |
| Probe6 | 6 | 5'PO4 | 3'H | GCTGAGTCGGAGACACGCAG GGATGAGACGCCACAACA**GG CC**TTCAGGCTCTCGCTGTGC CCAATATTGTCTTTGTGTTC CCGGACATAGTCCAG |
| Probe7 | 7 | 5'PO4 | 3'PO$_4$ | GCTGAGTCGGAGACACGCAG GGATGAGACGCCACAACA**GG CC**TTCAGGCTCTCGCTGTGC CCAATATTGTCTTTGTGTTC CCGGACATAGTCCAG |

(continued)

| Name | SEQ ID NO. | 5'-end modification | 3'-end modification | Sequence (5'→3') |
|------|-----------|---------------------|---------------------|------------------|
| Probe8 | 8 | 5'PO4 | 2'O-Methyl | <u>GCTGAGTCGGAGACACGCAG GGATGAGACGCCACAACA**GG CCTT**CAGGCTCTCGCTGTGC</u> CCAATATTGTCTTTGTGTTC CCGGACATAGTCCAG |
| Probe9 | 9 | 5'App | 3'C3 Spacer | <u>GCTGAGTCGGAGACACGCAG GGATGAGACGCCACAACA**GG CCTT**CAGGCTCTCGCTGTGC</u> CCAATATTGTCTTTGTGTTC CCGGACATAGTCCAG |
| Probe10 | 10 | 5'PO4 | 3'C3 Spacer | <u>GCTGAGTCGGAGACACGCAG GGATGAGACGCCACAACA**GG CCTT**CAGGCTCTCGCTGTGC</u> CCAATATTGTCTTTGTGTTC CCGGACATA**rG**TCCT |
| Probe11 | 11 | 5'PO4 | 3'C3 Spacer | <u>GCTGAGTCGGAGACACGCAG GGATGAGACGCCACAACA**GG CCTT**CAGGCTCTCGCTGTGC</u> CCAATATTGTCTTTGTGTTC CCGGACATAGTCCAGT |
| UF | 12 | 5'OH | 3'OH | CCTTCAGGCTCTCGCTGTGC |
| UR | 13 | 5'OH | 3'OH | CCTGTTGTGGCGTCTCATCC |
| F1 | 14 | 5'OH | 3'OH | TCCCGGACAT AGTCCAGGAGGC |
| RX1 | 15 | 5'OH | 3'OH | CGTGTGCCGCCTGCTGGG |
| MX1 | 16 | 5'FAM | 3'MGB | CACGGTGGAGGTGAGGC |
| REF | 17 | 5'OH | 3'OH | GATCCTTCAGGCTCTCGCTG TGCCCAATATTGTCTTTGTG TTCCCGGACATAGTCCAGGA GGCAGCCGAAGGGCATGAGC TGCGTGATGAGCTGCACGGT GGAGGTGAGGCAGATGCCCA GCAGGCGGCACACGTGGGGG TTGCTGAGTCGGAGACACGC AGGGATGAGACGCCACAACA GGATC |

All of the probes in this example contain universal sequence (underlined). All oligonucleotides were synthesized by Shanghai BIOLIGO Biological Co., Ltd. After purified twice by HPLC, the purity of the probe was over 99%. The specific sequences of the probes of SEQ ID NO. 1-11 in the table are all directed against the 20th exon region of the EGFR gene.

The modifications of the 5'-end nucleotides and the 3'-end nucleotides of each probe are shown in Table 1. Among them, the 3-position hydroxyl group of the 3'-end nucleotide of probe 6 (SEQ ID NO.6) was replaced by a hydrogen atom and became a dideoxynucleotide; the 2-position hydrogen atom of the 3'-end nucleotide of probe 8 (SEQ ID NO.8) was replaced by a methoxy group; the 5-position hydroxyl group of the 5'-end nucleotide of probe 9 (SEQ ID NO.9) was replaced by adenosine; the deoxyribonucleotide G near the 3'-end in the sequence of probe10 (SEQ ID NO.10) was replaced by a ribonucleotide rG; the last base of both probe 10 and probe 11 (SEQ ID NO. 10, SEQ ID NO. 11) are mismatched bases.

UF and UR (SEQ ID NO.12, SEQ ID NO.13) are the front and back primers used for PCR amplification of circularized products, F1 (SEQ ID NO.17) is the front primer used to detect the EGFR gene exon 20 region, RX1 and MX1 (SEQ ID NO. 19 and SEQ ID NO. 20) are post-primers and MGB probes used to detect the EGFR gene exon 20 region, respectively.

## MAIN REAGENTS AND MATERIALS

Cell DNA extraction kit and RNA Clean Kit for purification of single-stranded amplification products were purchased from Tiangen Biochemical Technology (Beijing) Co., Ltd.. rTaq DNA polymerase, KOD plus high-fidelity DNA polymerase and reaction master mix(QPK-101) for PCR detection were purchased from TOYOBO, Japan. RNAse $H_2$ thermostable nuclease was purchased from IDT Company, USA. APG ssDNA ligase, APG Enchant high-fidelity DNA polymerase were purchased from Shanghai Apogenomics Biotechnology Co., Ltd.. USER enzyme, Q5 high-fidelity DNA polymerase, T4 RNA ligase and 5'App DNA/RNA thermostable ligase were purchased from NEB Company, USA. Agencourt AMPure magnetic beads were purchased from Beckman Company, USA. The calibrator used for quantitative detection in the relevant process was synthesized by Shanghai BIOLIGO Biotechnology Co., Ltd. according to the REF sequence, and was prepared in 20% glycerol TE buffer according to a certain concentration gradient.

## Method

### Step 1. Sample preparation

The gDNA was extracted from the blood cell samples of healthy volunteers by using a cell DNA extraction kit, and fragmented by ultrasound into fragments with an average length of about 150 bp. Set aside for use after quantitative determination by qubit. Deionized water was used as a control sample NC.

### Step 2. Quantitative detection of samples

Prepare a PCR quantitative detection system for the sample according to Table 2, and detect quantitatively the copy number concentration of the sample using the detection system for the EGFR20 exon region according to the amplification conditions in Table 3.

| Table 2. PCR quantitative detection system of samples | | |
| --- | --- | --- |
| Compositions | Dosage($\mu$L) | Final Concentration |
| 2×Taqman Master Mix (Toyobo) | 25 | 1× |
| F1 (10 $\mu$M) | 1.5 | 300 nM |
| RX1 (10 $\mu$M) | 1.5 | 300 nM |
| MX1 (10 $\mu$M) | 0.5 | 100 nM |
| Deionized water | 17.5 | / |
| All samples/calibrators | 4 | / |
| Total Volume | 50 | / |

| Table 3 Amplification conditions for PCR quantitative detection | | |
| --- | --- | --- |
| Cycles | Temperature | Time |
| 1 cycle | 95 °C | 3 min |
| 45 cycles | 95 °C | 10 sec |
| | 60 °C | 30 sec |

### Step 3. Linear Amplification

Prepare linear amplification reaction system according to Table 4, Table 5 and Table 6, respectively. The PCR procedure for linear amplification is shown in Table 7.

| Table 4 High-fidelity linear amplification reaction system | | |
|---|---|---|
| Compositions | Volume(μL) | Final Concentration/Total copies |
| Deionized water | 3.1 | / |
| 5X Buffer | 5 | 1× |
| probe (1 μM) | 0.5 | 50 nM |
| dNTP (2 mM) | 1.25 | 100 μM |
| APG Enchant high-fidelity polymerase (2U/μL) | 0.15 | |
| Samples | 15 | 20000 copies |
| Total | 25 | / |

| Table 5 General linear amplification reaction system | | |
|---|---|---|
| Compositions | Volume(μL) | Final Concentration/Total copies |
| Deionized water | 4 | / |
| 10 × Taq Buffer | 2.5 | 1× |
| MgCl$_2$ | 1.5 | 1.5 mM |
| probe (1 μM) | 0.5 | 50 nM |
| dNTP (2 mM) | 1.25 | 100 μM |
| rTaq polymerase (2.5U/μL) | 0.25 | |
| Samples | 15 | 20000 copies |
| Total | 25 | / |

| Table 6. Nuclease-containing linear amplification re action system | | |
|---|---|---|
| Compositions | Volume(μL) | Final Concentration/Total copies |
| Deionized water | 3.8 | / |
| 10 × Taq Buffer | 2.5 | 1× |
| MgCl$_2$ | 1.5 | 1.5 mM |
| probe (1 μM) | 0.5 | 50 nM |
| dNTP (2 mM) | 1.25 | 100 μM |
| rTaq polymerase (2U/uL) | 0.25 | |
| RNAse H$_2$ Nuclease | 0.2 | |
| Samples | 15 | 20000 copies |
| Total | 25 | / |

| Table 7. Linear Amplification Procedure | | |
|---|---|---|
| Cycles | Temperature | Time |
| 1 cycle | 98 °C | 2 min |

(continued)

| Table 7. Linear Amplification Procedure | | |
|---|---|---|
| Cycles | Temperature | Time |
| 1/20/40/80 cycles | 98 °C | 15 sec |
| | 70 °C | 45 sec |
| 1 cycle | 72 °C | 4 min |

3.2 After the linear amplification procedure is completed, purify the single-stranded amplified product by RNA Clean Kit to remove the unextended probe, and elute with 30 µL of elution buffer.

3.3 Linear Amplification Efficiency Detection

The PCR detection system of the linear amplified product is shown in Table 2, and the PCR procedure for detecting the linear amplification is shown in Table 3.

**Step 4. Circularization ligation**

According to the results of step 3, the partial linear amplified product obtained in step 2 and the control samples QC were used for circularization reaction according to Table 8, Table 9 and Table 10, respectively.

| Table 8. APG ssDNA ligase circularization system | | | |
|---|---|---|---|
| Compositions | Volume(µL) | Final Concentration | Ligation Conditions |
| 10X ssDNA ligation Buffer | 4 | 1X | 60°C 1hr, 95°C 10 min, inactivating the ligase |
| 50 mM MnCl$_2$ | 2 | 2.5 mM | |
| APG ssDNA ligase (200 U/µL) | 1 | 5 U/µL | |
| Linear amplified product | 25 | | |
| Deionized water | 8 | | |
| Total | 40 | / | |

| Table 9. T4RNA ligase circularization system | | | |
|---|---|---|---|
| Compositions | Volume(µL) | Final Concentration | Ligation Conditions |
| 10X ligation Buffer | 4 | 1X | 16°C 4hr, 60°C 15min, inactivating the ligase |
| T4 RNA ligase | 2 | 5 U / µL | |
| Deionized water | 1 | | |
| Linear amplified product | 25 | | |
| 50% PEG | 8 | 10% | |
| Total | 40 | / | |

| Table 10. 5'App thermostable ligase circularization system | | | |
|---|---|---|---|
| Compositions | Volume($\mu$L) | Final Concentration | Ligation Conditions |
| 10X NE Buffer 1 | 4 | 1X | 65°C 1hr, 95°C 15min, inactivating the ligase |
| 50 mM MnCl$_2$ | 2 | 2.5 mM | |
| 5'App DNA/RNA thermostable ligase (100 U/$\mu$L) | 2 | 5 U/$\mu$L | |
| Linear amplified product | 25 | | |
| 50% PEG | 8 | 10% | |
| Total | 41 | / | |

**Step 5. Detection of circularized product yield**

The circularized product was diluted 10 times with TE buffer, and a PCR detection system was prepared according to Table 11 to detect the yield of the target circularized product in step 4, and at the same time, the control samples NC without template was detected. The PCR procedure is shown in Table 12. The target circularized product molecules were quantified with the calibrator to evaluate the yield of the target circularized product.

| Table 11. Detection system for target ci rcularized product | | |
|---|---|---|
| Reagents | Dosage($\mu$L) | Final Concentration |
| 2 $\times$ Taqman master Mix | 12.5 | 1$\times$ |
| F1 (10 $\mu$M) | 0.75 | 300 nM |
| UR (10 $\mu$M) | 0.75 | 300 nM |
| MX1 (10 $\mu$M) | 0.25 | 100 nM |
| Deionized water | 8.75 | / |
| Calibrators / Circularized product | 2 | / |
| Total | 25 | / |

| Table 12. PCR detection conditions for target circularized product | | |
|---|---|---|
| Cycles | Temperature | Time |
| 1 cycle | 95 °C | 3 min |
| 45 cycles | 95 °C | 10 sec |
| | 60 °C | 30 sec |

**Step 6. Amplifying the circularized product**

PCR amplification was performed on the circularized product obtained in step 4 according to the reaction system in Table 13 and the procedure in Table 14.

| Table 13. Circularized amplified system | | |
|---|---|---|
| Compositions | Volume($\mu$L) | Final Concentration |
| Circularized product | 30 | / |
| 5$\times$ Q5 Buffer | 10 | 1X |
| dNTP (10 mM each) | 1 | 0.2 mM |

(continued)

| Table 13. Circularized amplified system | | |
|---|---|---|
| Compositions | Volume($\mu$L) | Final Concentration |
| Q5 DNA polymerase (2 U/$\mu$L) (NEB) | 1 | / |
| UF (10 $\mu$M) | 1 | 200 nM |
| UR (10 $\mu$M) | 1 | 200 nM |
| Deionized water | 6 | / |
| Total | 50 | / |

| Table 14. Library Amplification PCR Procedure | | |
|---|---|---|
| Cycles | Temperature | Time |
| 1 cycle | 98 °C | 3 min |
| 15 cycles | 98 °C | 10 sec |
| | 72 °C | 30 sec |
| 1 cycle | 72 °C | 5 min |

After the reaction is completed, purify the amplified library with 80 $\mu$L of AMPure XP magnetic beads according to the operation instructions, and finally wash the purified product in 20 $\mu$L of elution buffer (10 mM Tris-HCl, 0.1 mM EDTA, pH 8.0). Transfer the supernatant to a new microcentrifuge tube on a magnetic stand. So far, the molecular expansion of the targeted library containing the universal sequence is completed.

After the amplified library product was diluted 100 times with TE buffer, the library product was detected using the detection system and conditions shown in Table 11 and Table 12.

**Experimental results and analysis**

In this example, four parameters of linear amplification multiple, circularization ligation efficiency, molecular conversion rate and library expansion multiple are used to evaluate the technical effect of the method and system of the present invention on the amplification and ligation of the target region. The detailed calculation method is as follows:

Linear amplification multiple = output copy number of target gene after linear amplification / input copy number of target gene before linear amplification

The yield of circularized molecules = output copy number of the circularized amplified product of the target gene / input copy number of the linear amplified product of the target gene $\times$ 100%

Molecular transformation rate = output copy number of circularized product of target gene / input copy number before amplification $\times$ 100%

Library expansion multiple = copy number after expansion and purification / output copy number of circularized product $\times$ 100%

**Results 1. The effect of different types of polymerases on the linear amplification efficiency of differently-modified probes is shown in Table 15**

| Table 15. Linear amplification efficiency of differently-modified probes | | | | | |
|---|---|---|---|---|---|
| Probes | Types of DNA polymerase | Nuclease | Cycles | Output copy number after linear amplification | Input copy number before amplification | Linear amplification multiple |
| Probe 1 | High-fidelity polymerase | None | 40 | 543000 | 20000 | 27.2 |
| Probe2 | High-fidelity polymerase | None | 40 | 521000 | 20000 | 26.1 |
| Probe3 | high-fidelity polymerase | None | 40 | 384000 | 20000 | 19.2 |
| Probe4 | high-fidelity polymerase | None | 40 | 416000 | 20000 | 20.8 |
| Probe5 | high-fidelity polymerase | None | 40 | 69300 | 20000 | 3.5 |
| Probe6 | high-fidelity polymerase | None | 40 | 84200 | 20000 | 4.2 |
| Probe7 | high-fidelity polymerase | None | 40 | 64000 | 20000 | 3.2 |
| Probe8 | high-fidelity polymerase | None | 40 | 92000 | 20000 | 4.6 |
| Probe9 | high-fidelity polymerase | None | 40 | 392000 | 20000 | 19.6 |
| Probe10 | high-fidelity polymerase | None | 40 | 408000 | 20000 | 20.4 |
| Probe11 | high-fidelity polymerase | None | 40 | 286000 | 20000 | 14.3 |
| Probe1 | common polymerase | None | 40 | 623000 | 20000 | 31.2 |
| Probe2 | common polymerase | None | 40 | 598000 | 20000 | 29.9 |
| Probe3 | common polymerase | None | 40 | 23100 | 20000 | 1.2 |

(continued)

| Probe4 | common polymerase | None | 40 | 29200 | 20000 | 1.5 |
|---|---|---|---|---|---|---|
| Probe5 | common polymerase | None | 40 | 31300 | 20000 | 1.6 |
| Probe6 | common polymerase | None | 40 | 32800 | 20000 | 1.6 |
| Probe7 | common polymerase | None | 40 | 27000 | 20000 | 1.4 |
| Probe8 | common polymerase | None | 40 | 56000 | 20000 | 2.8 |
| Probe9 | common polymerase | None | 40 | 21200 | 20000 | 1.1 |
| Probe10 | common polymerase | None | 40 | 23800 | 20000 | 1.2 |
| Probe11 | common polymerase | None | 40 | 22400 | 20000 | 1.1 |
| Probe10 | common polymerase | Yes | 40 | 432000 | 20000 | 21.6 |
| Probe11 | common polymerase | Yes | 40 | 18600 | 20000 | 0.9 |

For all control samples, no signal was detected.

The linear amplification reaction systems and conditions involved in Table 15 are shown in Tables 4 to 7. In high-fidelity polymerase reaction system, APG Enchant high-fidelity DNA polymerase with 3'-5' exonuclease activity was used, while what is used in common polymerase reaction system is rTaq enzyme with 5'-3' exonuclease activity but not 3'-5' exonuclease activity. In order to achieve the function of exonuclease, we added a thermostable nuclease into the common polymerase reaction system, it can also excise the block-modified 3'-end and achieve a technical effect similar to that of using high-fidelity DNA polymerase.

It can be seen from the test results that:

1) After the probe with blocking-modified 3'-end nucleotide binds to the template under the action of high-fidelity polymerase, the blocked group is excised and the probe is activated, so that the target sequence can be effectively amplified.
2) The combination of thermostable nuclease and common polymerase rTaq can also activate the probes modified by ribonucleotide groups, which enhances the applicability of the enrichment method of the present invention.
3) Different substituent groups have obvious differences in the linear amplification effect. The effect of replacing the 3'-end hydroxyl group of the probe with C3 Spacer is ideal, which has obvious advantages compared with other substituent groups. The data from result 2 in this example are mainly the result data obtained from the probe in which the 3'-end hydroxyl group was replaced with C3 Spacer.
4) When there is a mismatch near the 3'-end of the probe whose 3'-end nucleotide was blocking-modified (Probe 11), it has no significant effect on the linear amplification reaction driven by high-fidelity polymerase with proofreading function, otherwise it is helpful.

**Results 2.** The effects of different amplification cycle numbers on the linear amplification efficiency are shown in Table 16.

See step 2 for the linear amplification reaction system and conditions involved in Table 16, and see step 3 for the linear amplification efficiency detection method.

| Table 16. Amplification results at different cycle numbers (QC Samples) | | | | | | |
|---|---|---|---|---|---|---|
| Probes | Type of DNA polymerase | Nuclease | Cycles | Output copy number after linear amplification | Input copy number before amplification | Linear amplification multiple |
| Probe1 | high-fidelity polymerase | None | 80 | 922000 | 20000 | 46.1 |
| Probe2 | high-fidelity polymerase | None | 80 | 893400 | 20000 | 44.7 |
| Probe4 | high-fidelity polymerase | None | 80 | 635000 | 20000 | 31.8 |

(continued)

| Table 16. Amplification results at different cycle numbers (QC Samples) | | | | | | |
|---|---|---|---|---|---|---|
| Probes | Type of DNA polymerase | Nuclease | Cycles | Output copy number after linear amplification | Input copy number before amplification | Linear amplification multiple |
| Probe11 | high-fidelity pol ymerase | None | 80 | 742000 | 20000 | 37.1 |
| Probe10 | common pol ymerase | Yes | 80 | 821000 | 20000 | 41.1 |
| Probe1 | high-fidelity pol ymerase | None | 40 | 543000 | 20000 | 27.2 |
| Probe2 | high-fidelity pol ymerase | None | 40 | 521000 | 20000 | 26.1 |
| Probe4 | high-fidelity pol ymerase | None | 40 | 416000 | 20000 | 20.8 |
| Probe11 | high-fidelity pol ymerase | None | 40 | 286000 | 20000 | 14.3 |
| Probe10 | common pol ymerase | Yes | 40 | 432000 | 20000 | 21.6 |
| Probe1 | high-fidelity pol ymerase | None | 20 | 286000 | 20000 | 14.3 |
| Probe2 | high-fidelity polymerase | None | 20 | 263000 | 20000 | 13.2 |
| Probe4 | high-fidelity polymerase | None | 20 | 221000 | 20000 | 11.1 |
| Probe11 | high-fidelity pol ymerase | None | 20 | 216000 | 20000 | 10.8 |
| Probe10 | common polymerase | Yes | 20 | 258000 | 20000 | 12.9 |
| Probe1 | high-fidelity polymerase | None | 1 | 33600 | 20000 | 1.7 |
| Probe2 | high-fidelity polymerase | None | 1 | 32800 | 20000 | 1.6 |
| Probe4 | high-fidelity polymerase | None | 1 | 30300 | 20000 | 1.5 |
| Probe11 | high-fidelity pol ymerase | None | 1 | 29800 | 20000 | 1.5 |
| Probe10 | common polymerase | Yes | 1 | 31600 | 20000 | 1.6 |

The test results showed that both the high-fidelity polymerase system and the common polymerase plus nuclease system can effectively improve the linear amplification multiple of the 3'- end blocked probe by increasing the number of cycles.

**Results 3. The effect of blocked probes on the circularization results is shown in Table 17**

See step 4 for the circularization reaction system involved in Table 17, and see step 5 for the method of detecting the ligation efficiency.

| Table 17 Test results after circularization by different probes | | | | | | | |
|---|---|---|---|---|---|---|---|
| Probes | Enzyme for linear amplification | Cycles | Ligase for circularization | Input copy number after linear amplification | Copy number of target circularized molecule | Yield of target circularized molecule | Molecular conversion rate |
| Probe1 | high-fidelity polymerase | 80 | APG ssDNA ligase | 768333 | 0 | 0.00% | 0.00% |
| Probe2 | high-fidelity polymerase | 80 | APG ssDNA ligase | 744500 | 521 | 0.07% | 2.61% |
| Probe4 | high-fidelity polymerase | 80 | APG ssDNA ligase | 529167 | 28734 | 5.43% | 143.67% |
| Probe11 | high-fidelity polymerase | 80 | APG ssDNA ligase | 618333 | 29124 | 4.71% | 145.62% |
| Probe10 | common polymerase + nuclease | 80 | ssDNA ligase | 684167 | 21414 | 3.13% | 107.07% |

The results showed that: 1) Using the probe whose hydroxyl group on 3'-end nucleotide was replaced by C3 Spacer, the ideal yield of target circularized molecule and molecular conversion rate can be obtained, and the latter can exceed 100%, indicating that the enrichment method of the present invention can achieve complete lossless from the original molecule to the library molecules configured with sequencing universal sequences at both ends;

2) After the unmodified probe system reacted, only a very small amount of target circularized molecule can be detected;

3) Only after the 5'-end nucleotide was modified by phosphorylation, the probe can achieve self-circularization under the action of APG ssDNA ligase;

4) For probes blocking-modified by ribonucleotide near the 3'-end, a higher yield of target circularized molecules can also achieve by using the system of common polymerase plus nuclease.

**Results 4. The effects of probes with different 5'-end modifications and different ligases on the circularization efficiency are shown in Table 18.**

See Table 8, Table 9 and Table 10 for the ligation reaction system involved in Table 18, and step 5 for the detection method of circularization efficiency.

| Table 18. Test results for different ligases and probes with different modification on 5'-end | | | | | | | |
|---|---|---|---|---|---|---|---|
| Probes | Enzymes for linear amplification | Cycles | Circularization ligase | Copy number of input molecular after linear amplification | Copy number of target circularized molecule | Yield of target circularized molecule | Molecular conversion rate |
| Probe4 | high-fidelity polymerase | 80 | ssDNA ligase | 529167 | 28734 | 5.43% | 143.67% |
| Probe4 | high-fidelity polymerase | 80 | T4 RNA ligase | 529167 | 2963 | 0.56% | 14.82% |
| Probe9 | high-fidelity polymerase | 80 | 5'AppDNA/RNA ligase | 504680 | 7318 | 1.45% | 36.59% |

The results showed that the use of 5'-phosphorylated blocking probes combined with APG ssDNA ligase has an ideal circularization effect, which is better than that of adenosine-modified blocking probes combined with other ligases.

**Results 5. The detection of library expansion efficiency of ligated products is shown in Table 19.**

| Table 19 Test results after circularization of different probes | | | | | | | |
|---|---|---|---|---|---|---|---|
| Probes | Enzymes for linear amplification | Cycles | Circularization ligase | Input copy number after linear amplification | Copy number of circularized molecule | Copy number of target molecule after library expansion | Library expansion multiple |
| Probe2 | high-fidelity polymerase | 80 | ssDNA ligase | 744500 | 521 | 1459 | 2.8 |
| Probe4 | high-fidelity polymerase | 80 | ssDNA ligase | 529167 | 28734 | 3600339 | 125.3 |
| Probe11 | high-fidelity polymerase | 80 | ssDNA ligase | 618333 | 29124 | 3145338 | 108.0 |
| Probe10 | common polymerase+ nuclease | 80 | ssDNA ligase | 684167 | 21414 | 1893034 | 88.4 |

See step 6 for the library expansion reaction system and procedure involved in Table 19.
The results showed that the target circularized molecule of the blocking probe can be well amplified. The library expansion multiple is about 100 times, which is significantly better than that of unblocked probes.

**Conclusions:**

The blocking modification of the hydroxyl group of nucleotide at the 3'-end of the probe is very important, which can prevent the self-ligation of the probe and greatly improve the ligation efficiency of the target molecule. The probe after combined to the template can be subjected to multiple cycles of linear amplification after the modified group is excised by DNA polymerase. In addition, the use of thermostable APG ssDNA ligase, the purification treatment after circularization, and the use of universal primers for amplification after circularization can also improve the purity and detection sensitivity of the library product.

**Example 2**

The oligonucleotides used in the examples are shown in Table 20:

Table 20.

| Name | SEQ ID NO. | 5'-end modification | 3'-end modification | Sequence (5'→3') |
|---|---|---|---|---|
| Probe4 | 4 | 5'PO4 | 3'C3 Spacer | GCTGAGTCGGAGACACGCAG GGATGAGACGCCACAACAGG CCTTCAGGCTCTCGCTGTGC CCAATATTGTCTTTGTGTTC CCGGACATAGTCCAG |

(continued)

| Name | SEQ ID NO. | 5'-end modification | 3'-end modification | Sequence (5'→3') |
|---|---|---|---|---|
| Probe 12 | 18 | 5'PO4 | 3'C3 Spacer | GCTGAGTCGGAGACACGCAG GGATGAGACGCCACAACAGG CCUTCAGGCTCTCGCTGTGC CCAATATTGTCTTTGTGTTC CCGGACATAGTCCAG |
| UF | 12 | 5'OH | 3'OH | CCTTCAGGCTCTCGCTGTGC |
| UR | 13 | 5'OH | 3'OH | CCTGTTGTGGCGTCTCATCC |
| F1 | 14 | 5'OH | 3'OH | TCCCGGACAT AGTCCAGGAGGC |
| RX1 | 15 | 5'OH | 3'OH | CGTGTGCCGCCTGCTGGG |
| MX1 | 16 | 5'FAM | 3'MGB | CACGGTGGAGGTGAGGC |
| REF | 17 | 5'OH | 3'OH | GATCCTTCAGGCTCTCGCTG TGCCCAATATTGTCTTTGTG TTCCCGGACATAGTCCAGGA GGCAGCCGAAGGGCATGAGC TGCGTGATGAGCTGCACGGT GGAGGTGAGGCAGATGCCCA GCAGGCGGCACACGTGGGGG TTGCTGAGTCGGAGACACGC AGGGATGAGACGCCACAACA GGATC |

Same as in Example 1, the probes in this example also contain universal sequences. After the probe was purified twice by HPLC, the purity was 99% or above.

Among them, the modification at both ends of probe 12 (SEQ ID NO. 18) is same as that of probe 4, and one base T is replaced by U in the general sequence. The other detection primers and probes are same as those in Example 1.

**Main reagents and materials**

APG ssDNA endonuclease was purchased from Shanghai Apogenomics Biotechnology Co., Ltd., and USER enzyme was purchased from NEB Company, USA, the other reagents and materials were same as in Example 1.

**Experimental methods**

The preparation of samples, the quantitative detection of samples, linear amplification and circularization ligation in this example are same as those in Example 1, wherein the initial template is 20,000 copies, APG Enchant high-fidelity polymerase was used for linear amplification, 80 cycles, and APG ssDNA ligase was used in circularization ligation.

USER enzyme or APG ssDNA endonuclease was added into the circularized product, and excise the circularized product at 37 degrees for 1 hour, and the unexcised circularized product was used as a control. APG ssDNA endonuclease can specifically excise single-stranded DNA containing 5' GGCC 3' in the sequence. After excised, the excised product was purified with RNA Clean Kit and eluted with 35 μL of elution buffer.

Take 5 μL of the eluted product and dilute it 10 times using TE buffer, and detect the yield of the target circularized product using the PCR detection system in Table 11 in Example 1. The PCR procedure is shown in Table 12.

According to step 6 in Example 1, the reaction system in Table 13 and the procedure in Table 14, PCR amplification was performed on the eluted product obtained after circularization in step 4.

After the reaction was finished, 80 μL of AMPure XP magnetic beads were taken to purify the amplified library. According to the operation instructions, the purified product was finally eluted in 20 μL of elution buffer (10 mM TrisCl, 0.1 mM EDTA, pH 8.0), and the supernatant was transferred from the magnetic stand to a new microcentrifuge tube. So far, the library expansion of the target library molecule containing universal sequence was completed.

After the amplified library products were diluted 100 times with TE buffer, the library products were detected using the detection systems and conditions in above Table 11 and Table 12.

**Experimental results and analysis**

Same as Example 1, this example also uses the four parameters of linear amplification multiple, circularized molecule yield, molecular conversion rate and library expansion multiple to evaluate the technical effect of this method on the amplification and ligation of the target region, and see example 1 for detailed calculation method.

Results 2.1 The influence of enzyme-digestion after circularization on detection is shown in Table 21.

Table 21. Influence of enzyme-digestion after circularization on detection

| Probes | Enzymes for linear amplification | Cycles | Enzyme-digestion after circularization | Input molecular copy number after linear amplification | Target circularized molecule copy number | Yield of circularized molecule | Molecular conversion rate |
|---|---|---|---|---|---|---|---|
| Probe4 | high-fidelity polymerase | 80 | None | 568000 | 38170 | 6.72% | 190.85% |
| Probe4 | high-fidelity polymerase | 80 | ssDNA endonuclease | 568000 | 105818 | 18.63% | 529.09% |
| Probe12 | high-fidelity polymerase | 80 | None | 375000 | 20775 | 5.54% | 103.88% |
| Probe12 | high-fidelity polymerase | 80 | USER enzyme | 375000 | 48638 | 12.97% | 243.19% |

The results showed that after the circularized product was excised by enzyme, the detected yield of circularized molecule and molecular conversion rate were significantly improved.

**Results 2.2 The effect of enzyme-digestion after circularization on library expansion efficiency is shown in Table 22.**

| Table 22. Effect of enzyme-digestion after circularization on library expansion efficiency | | | | | | |
|---|---|---|---|---|---|---|
| Probes | Enzymes for linear amplification | Cycles | Enzyme-digestion after circularization | Copy number of target circularized molecule | Copy number of target molecule after library expansion | Library expansion multiple |
| Probe4 | high-fidelity polymerase | 80 | None | 38170 | 12748646 | 334 |
| Probe4 | high-fidelity polymerase | 80 | ssDNA endonuclease | 105818 | 101162390 | 956 |
| Probe12 | high-fidelity polymerase | 80 | None | 20775 | 5152200 | 248 |
| Probe12 | high-fidelity polymerase | 80 | USER enzyme | 48638 | 38229075 | 786 |

The results showed that the library expansion efficiency was significantly improved after the circularized product was excised by enzyme.

**Example 3**

This example evaluated the ability to detect rare molecules and rare variant molecules of tiny fragments after linear amplification and circularization library construction of real samples using the enrichment method and system of the present invention.
The oligonucleotide sequences used in this example were shown in Table 23:

Table 23

| Name | SEQ ID NO. | 5'-end modification | 3'-end modification | Sequence (5'→3') |
|---|---|---|---|---|
| Probe4 | 4 | 5'PO4 | 3'C3 Spacer | GCTGAGTCGGAGACACGCAGGGATGAGACGCCACAACAGGCCTTCAGGCTCTCGCTGTGCCCAATATTGTCTTTGTGTTCCCGGACATAGTCCAG |
| Probe 13 | 19 | 5'PO4 | 3'C3 Spacer | GCTGAGTCGGAGACACGCAGGGATGAGACGCCACAACAGGCCTTCAGGCTCTCGCTGTGCGCTGCCGAAGAATTGCAGTTTGCTTCCCGC |
| FX1 | 20 | 5'OH | 3'OH | GCGTGATGAGT |

(continued)

| Name | SEQ ID NO. | 5'-end modification | 3'-end modification | Sequence (5'→3') |
|------|-----------|--------------------|--------------------|------------------|
| FX2 | 21 | 5'OH | 3'OH | CGCTTCGGTACTCTGC |
| RX2 | 22 | 5'OH | 3'OH | GTCATCCCTGTACAACCTG |
| MX2 | 23 | 5'FAM | 3'MGB | GCGAAGTGCAACTGGACAA |
| UM | 24 | 5'FAM | 3'MGB | TGCGTGTCTCCGACTCAGC |
| UF | 12 | 5'OH | 3'OH | CCTTCAGGCTCTCGCTGTGC |
| UR | 13 | 5'OH | 3'OH | CCTGTTGTGGCGTCTCATCC |
| RX1 | 15 | 5'OH | 3'OH | CGTGTGCCGCCTGCTGGG |
| MX1 | 16 | 5'FAM | 3'MGB | CACGGTGGAGGTGAGGC |

The specific sequence of probe 13 (SEQ ID NO. 19) targets the SRY gene on sex chromosome Y. FX1 (SEQ ID NO. 20) is a pre-primer used to specifically detect SNP-Q787 (2361 G>A) in the $20^{th}$ exon region of the EGFR gene. In the previous example, the pairing of RX1 and MX1 constituted a PCR detection system for the specific detection of SNP-Q787. FX2, RX2 and MX2 (SEQ ID NO. 21-23) are the pre-primer, post-primer and MGB probe for detecting SRY probe amplification products, respectively. UM (SEQ ID NO. 24) is an MGB probe used to detect the universal sequence.

**Main reagents and materials**

Cell-free DNA extraction kits were purchased from Shanghai Zensight Gene Technology Co., Ltd., and others were the same as in Examples 1 and 2.

**EXPERIMENTAL METHODS**

**STEP 1. Preparation of target library**

The cfDNA in healthy human plasma samples was extracted using a cell-free DNA extraction kit, and quantified using Qubit, then set aside for later use. Fragments of the exon region of EGFR-20 were amplified by F1 and RX1. The status of SNP-Q787 was detected using a first-generation sequencing method. Male cfDNA samples homozygous for SNP were incorporated into female wild-type cfDNA samples at mass ratios of 1%, 0.1%, 0.03%, and 0.01%, while unincorporated female wild-type cfDNA samples were used as blank control (QC). 50ng (about 15000 copies, 15ul in total) was used as the total starting template amount for a single sample for subsequent targeted library construction. Two replicate wells were set for each gradient, and the average value of the results of the replicate wells was finally used as the detection result of this kind of sample.

Target library construction of samples were performed using APG ssDNA endonuclease. Probe 4 and probe 13 were used as the probe, and the concentration was 50nM. The cycle number of linear amplification was 80, APG ssDNA ligase was used for circularization, 12 cycles of amplification were performed after enzyme-digestion and purification, and then, 20 μL of library products were obtained after purification with AMPure XP magnetic beads.

**Step 2. qPCR detection of different specific primer/probe combinations**

The original samples and the targeted library samples were detected by tri-specific PCR detection system respectively, and then the targeted library samples were detected by bispecific and monospecific PCR. Take the average CT value of the detection samples, take the average CT value of the QC samples as the detection background, and use the difference ΔCT between the two as the evaluation standard. A significant difference was considered when ΔCT≥2.5.

The tri-specific, bispecific and monospecific detection systems of EGFR gene exon 20 Q787Q site were shown in Tables 24-26, respectively. The SRY gene tri-specific, bispecific, and monospecific detection systems were shown in Tables 27-29, respectively. The qPCR detection procedure was shown in Table 30.

Table 24. Q787Q site tri-specific detection system:

| Reagent | Dosage (μL) | Final concentration |
|---|---|---|
| 2×Taqman master Mix | 10 | 1× |
| FX1 (10 μM) | 0.6 | 300 nM |
| RX1 (10 μM) | 0.6 | 300 nM |
| MX1 (10 μM) | 0.2 | 100 nM |
| H$_2$O | 6.6 | / |
| Samples to be tested | 2 | / |
| Total | 20 | / |

Table 25. Q787Q site bispecific detection system:

| Reagent | Dosage (μL) | Final concentration |
|---|---|---|
| 2×Taqman master Mix | 10 | 1× |
| FX1 (10 μM) | 0.6 | 300 nM |
| UR (10 μM) | 0.6 | 300 nM |
| MX1 (10 μM) | 0.2 | 100 nM |
| H$_2$O | 6.6 | / |
| Samples to be tested | 2 | / |
| Total | 20 | / |

Table 26. Q787Q site monospecific detection system:

| Reagent | Dosage (μL) | Final concentration |
|---|---|---|
| 2×Taqman master Mix | 10 | 1× |
| FX1 (10 μM) | 0.6 | 300 nM |
| UR (10 μM) | 0.6 | 300 nM |
| UM (10μM) | 0.2 | 100 nM |
| H$_2$O | 6.6 | / |
| Samples to be tested | 2 | / |
| Total | 20 | / |

Table 27. SRY gene tri-specific detection system:

| Reagent | Dosage (μL) | Final concentration |
|---|---|---|
| 2×Taqman master Mix | 10 | 1× |
| FX2 (10 μM) | 0.6 | 300 nM |

(continued)

| Reagent | Dosage ($\mu$L) | Final concentration |
|---|---|---|
| RX2 (10 $\mu$M) | 0.6 | 300 nM |
| MX2 (10$\mu$M) | 0.2 | 100 nM |
| $H_2O$ | 6.6 | / |
| Samples to be tested | 2 | / |
| Total | 20 | / |

Table 28. SRY gene bispecific detection system:

| Reagent | Dosage ($\mu$L) | Final concentration |
|---|---|---|
| 2$\times$Taqman master Mix | 10 | 1 $\times$ |
| FX2 (10 $\mu$M) | 0.6 | 300 nM |
| UR (10 $\mu$M) | 0.6 | 300 nM |
| MX2 (10$\mu$M) | 0.2 | 100 nM |
| $H_2O$ | 6.6 | / |
| Samples to be tested | 2 | / |
| Total | 20 | / |

Table 29. SRY gene monospecific detection system:

| Reagent | Dosage ($\mu$L) | Final concentration |
|---|---|---|
| 2$\times$Taqman master Mix | 10 | Final 1$\times$ |
| FX2 (10 $\mu$M) | 0.6 | 300 nM |
| UR (10 $\mu$M) | 0.6 | 300 nM |
| UM (10$\mu$M) | 0.2 | 100 nM |
| $H_2O$ | 6.6 | / |
| Samples to be tested | 2 | / |
| Total | 20 | / |

Table 30.

| Cycles | Temperature | Time |
|---|---|---|
| 1 cycle | 95°C | 4 min |
| 40 cycles | 95°C | 10 s |
| | 61°C | 30 s |

**Experimental results and analysis**

The detection results of EGFR SNP-Q787 site and SRY gene for the original samples and the samples after targeted library construction using different specific systems were shown in Tables 31 and 32, respectively.

Table 31. EGFR SNP-Q787 site detection results under different incorporation ratios

| Type of samples | Incorporation ratios | PCR system | CT1 | CT2 | CT mean | $\triangle$CT |
|---|---|---|---|---|---|---|
| Original samples | 1% | tri-specific | 30.23 | 31.06 | 30.65 | 7.29 |
| | 0.10% | tri-specific | 34.35 | 35.56 | 34.96 | 2.98 |
| | 0.03% | tri-specific | 36.43 | 36.85 | 36.64 | 1.29 |
| | 0.01% | tri-specific | 37.94 | 37.61 | 37.78 | 0.16 |
| | QC | tri-specific | 38.34 | 37.52 | 37.93 | 0.00 |
| Library samples | 1% | tri-specific | 25.12 | 24.98 | 25.05 | 9.67 |
| | 0.10% | tri-specific | 28.35 | 29.65 | 29.00 | 5.72 |
| | 0.03% | tri-specific | 31.52 | 32.06 | 31.79 | 2.93 |
| | 0.01% | tri-specific | 32.84 | 33.52 | 33.18 | 1.54 |
| | QC | tri-specific | 34.42 | 35.02 | 34.72 | 0.00 |
| Library samples | 1% | bispecific | 24.02 | 24.26 | 24.14 | 10.16 |
| | 0.10% | bispecific | 27.42 | 27.58 | 27.50 | 6.80 |
| | 0.03% | bispecific | 30.16 | 29.54 | 29.85 | 4.45 |
| | 0.01% | bispecific | 31.25 | 31.69 | 31.47 | 2.83 |
| | QC | bispecific | 34.56 | 34.04 | 34.30 | 0.00 |
| Library samples | 1% | monospecific | 23.56 | 23.43 | 23.50 | 10.04 |
| | 0.10% | monospecific | 27.54 | 26.92 | 27.23 | 6.30 |
| | 0.03% | monospecific | 29.22 | 28.95 | 29.09 | 4.45 |
| | 0.01% | monospecific | 31.04 | 30.46 | 30.75 | 2.78 |
| | QC | monospecific | 33.24 | 33.82 | 33.53 | 0.00 |

Table 32. SRY gene detection results under different incorporation ratios

| Type of samples | Incorporation ratios | PCR system | CT1 | CT2 | CT mean | $\triangle$CT |
|---|---|---|---|---|---|---|
| Original samples | 1% | tri-specific | 31.41 | 31.20 | 31.31 | 8.69 |
| | 0.10% | tri-specific | 36.84 | 35.30 | 36.07 | 3.93 |
| | 0.03% | tri-specific | 37.38 | 39.15 | 38.26 | 1.74 |
| | 0.01% | tri-specific | 40.00 | 40.00 | 40.00 | 0.00 |
| | QC | tri-specific | 40.00 | 40.00 | 40.00 | 0.00 |
| Library samples | 1% | tri-specific | 24.59 | 25.00 | 24.79 | 15.21 |
| | 0.10% | tri-specific | 28.34 | 28.76 | 28.55 | 11.45 |
| | 0.03% | tri-specific | 30.94 | 30.28 | 30.61 | 9.39 |
| | 0.01% | tri-specific | 33.44 | 32.59 | 33.02 | 6.98 |
| | QC | tri-specific | 40.00 | 40.00 | 40.00 | 0.00 |
| Library samples | 1% | bispecific | 24.46 | 24.60 | 24.53 | 15.47 |
| | 0.10% | bispecific | 27.77 | 27.74 | 27.75 | 12.25 |
| | 0.03% | bispecific | 30.11 | 30.95 | 30.53 | 9.47 |
| | 0.01% | bispecific | 32.50 | 32.88 | 32.69 | 7.31 |
| | QC | bispecific | 40.00 | 40.00 | 40.00 | 0.00 |

(continued)

| Type of samples | Incorporation ratios | PCR system | CT1 | CT2 | CT mean | $\Delta$CT |
|---|---|---|---|---|---|---|
| Library samples | 1% | monospecific | 24.97 | 23.98 | 24.48 | 14.27 |
| | 0.10% | monospecific | 27.32 | 27.40 | 27.36 | 11.39 |
| | 0.03% | monospecific | 29.60 | 29.84 | 29.72 | 9.03 |
| | 0.01% | monospecific | 32.08 | 32.22 | 32.15 | 6.60 |
| | QC | monospecific | 38.22 | 39.28 | 38.75 | 0.00 |

**Conclusions:**

The experimental results showed that blocking of probe is very important to the circularization enrichment method of the present invention, which can significantly improve the detection rate of rare molecules in cfDNA, and can increase the detection rate of library molecules by more than 10 times. The detection rate of original samples is generally 0.1%, and the detection rate of library samples can reach 0.01%.

The traditional tri-specific gene detection system was compared with the bispecific system and the monospecific primer/probe system in this example. For the Q787 site of single base variation, the $\Delta$CT value of the monospecific system and the bispecific system is larger than that of the tri-specific system, and it has a higher effective resolution. For the specific gene SRY, the detection sensitivity of different specific primer/probe systems is high, and all of the three systems can reach 0.01%.

In summary, the blocking modification of the 3-position hydroxyl group in the nucleotide at the 3'-end of the probe is very important, which can prevent the self-ligation of the probe and greatly improve the circularization efficiency. After the modified group is excised from the probe bound to the template, the probe can perform multiple cycles of linear amplification, which minimizes the loss of the original molecule. In addition, the use of APG ssDNA ligase, the enzyme-digestion treatment after circularization, and the amplification using universal primers after circularization can also play a role in improving the purity and detection sensitivity of the library product, respectively/together to form the technical solution of the present invention.

**Claims**

1. An enrichment method for a gene target region, comprising:

   (1) Amplifying the fragmented DNA containing a target region by specific probe to provide a captured extension product, wherein the specific probe includes both sequences complementary to the target region of the fragmented DNA and sequences not complementary to the target region of the fragmented DNA, and both the 3'-end and 5'-end nucleotides of the specific probe are modified,
   (2) Adding ligase to the captured extension product provided in step (1) to provide a ligated product, wherein the ligated product includes a circular ligated product and a linear ligated product.

2. The enrichment method for a gene target region according to claim 1, wherein in step (1), the fragmented DNA includes double-stranded DNA, single-stranded DNA and cDNA, and the length of the fragmented DNA is 25-200 bp, and/or, the amplification system of step (1) further includes DNA polymerase and dNTP.

3. The enrichment method for a gene target region according to claim 2, wherein the DNA polymerase has 3'-5' exonuclease activity;
   and/or, the dNTP is further coupled with a labeling molecule, and the labeling molecule is preferably biotin.

4. The enrichment method for a gene target region according to claim 1, wherein the amplification system of the step (1) further comprises:

   an active substance, which is used to excise the 3'-end modification group of the specific probe after the specific probe binds to the target region of the fragmented DNA;
   preferably, the active substance is a nuclease.

5. The enrichment method for a gene target region according to claim 1, wherein the specific probe further includes a universal sequence that can be recognized by the sequencing system;

   and/or, the 3-position hydroxyl group at the 3'-end nucleotide of the specific probe is substituted;
   and/or, the 2-position methoxy group at the 3'-end nucleotide of the specific probe is substituted;
   preferably, the substituent group at the 3'-end of the specific probe is selected from a hydrogen atom, a C3 Spacer group, a C6 Spacer group, a phosphate group or an amino group;
   and/or, the 3'-end tail region of the specific probe comprises mismatched base;
   and/or, the 5-position hydroxyl group at the 5'-end nucleotide of the specific probe is substituted;
   preferably, the substituent group at the 5'-end of the specific probe is selected from a phosphate group or an adenosine group.

6. The enrichment method for a gene target region according to claim 1, wherein the ligation system in step (2) includes a single-stranded ligase, and the single-stranded ligase is preferably T4 RNA ligase or thermostable RNA ligase.

7. The enrichment method for a gene target region according to claim 1, wherein further comprises (3) Setting an enzyme-digestion site in a sequence that is not complementary to the target region on the specific probe, and adding an endonuclease after step (2) for excising a circular ligated product in the ligated product provided in step (2) at the enzyme-digestion site in order to provide a linear ligated product;
   preferably, the enzyme-digestion site is uracil, and the endonuclease is ssDNA endonuclease or USER enzyme.

8. The enrichment method for a gene target region according to claim 7, wherein, further comprises (4) after the step (2), PCR amplifying the ligated product provided in the step (2);

preferably, in the step (4), the PCR amplification primer has a sequence complementary to the specific probe but not complementary to the sequence of the target region,
more preferably, the sequence complementary to the specific probe is a sequencing universal sequence; and/or,

(5) after the step (3), PCR amplifying the ligated products provided in the steps (2) and (3); preferably, in the step (5), the PCR amplification primers have sequence complementary to the sequence on both sides of the enzyme-digestion site;

more preferably, the sequences complementary to the sequences on both sides of the enzyme-digestion site are sequencing universal sequences;
and/or, purification is carried out after any of the steps (1) to (5).

9. The enrichment method for a gene target region according to claim 8, wherein, further comprises (6) detecting the amplified product provided in step (4) or (5) using detection primer 1, detection primer 2 and probe 3, wherein at least one of the detection primer 1, detection primer 2 and probe 3 contains gene-specific sequences;

preferably, the detection primer 1 comprises a gene-specific sequence, and the detection primer 2 and the probe 3 comprise a universal sequence;
and/or, the detection primer 2 and/or probe 3 comprise gene-specific sequences, too;
preferably, the probe 3 comprises a labelling molecule, and the sequence of the probe 3 is not complementary to that of the detection primer 1 or 2.

10. The enrichment method for a gene target region according to claim 1, wherein the enrichment method for a gene target region is used for nucleic acid detection.

11. A system for enriching target region of fragmented DNA, comprising specific probes and ligases suitable for the enrichment method for a gene target region according to any one of claims 1 to 10.

12. The system for enriching target region of fragmented DNA according to claim 11, wherein further comprises one or more of the following components: dNTPs coupled to labeled molecules, DNA polymerases, nucleases, and endonuclease;

and/or, further comprises PCR amplification primers, the amplification primers have sequences that are at least partially complementary to the universal sequences of the specific probes;
and/or, further comprises detection primer 1, detection primer 2 and probe 3, at least one of which contains a gene-specific sequence.

**Amended claims under Art. 19.1 PCT**

1. An enrichment method for a gene target region, comprising:

(1) Amplifying the fragmented DNA containing a target region by specific probe to provide a captured extension product, wherein the specific probe includes both sequences complementary to the target region of the fragmented DNA and sequences not complementary to the target region of the fragmented DNA, and both the 3'-end and 5'-end nucleotides of the specific probe are modified,
(2) Adding ligase to the captured extension product provided in step (1) to provide a ligated product, wherein the ligated product includes a circular ligated product and a linear ligated product;
the amplification system of the step (1) further comprises:

an active substance, which is used to excise the 3'-end modification group of the specific probe after the specific probe binds to the target region of the fragmented DNA;
preferably, the active substance is a nuclease;
the 3-position hydroxyl group at the 3'-end nucleotide of the specific probe is substituted;
and/or, the 2-position methoxy group at the 3'-end nucleotide of the specific probe is substituted;
preferably, the substituent group at the 3'-end of the specific probe is selected from a hydrogen atom, a C3 Spacer group, a C6 Spacer group, a phosphate group or an amino group;

2. The enrichment method for a gene target region according to claim 1, wherein in step (1), the fragmented DNA includes double-stranded DNA, single-stranded DNA and cDNA, and the length of the fragmented DNA is 25-200 bp, and/or, the amplification system of step (1) further includes DNA polymerase and dNTP.

3. The enrichment method for a gene target region according to claim 2, wherein the DNA polymerase has 3'-5' exonuclease activity;
and/or, the dNTP is further coupled with a labeling molecule, and the labeling molecule is preferably biotin.

4. The enrichment method for a gene target region according to claim 1, wherein the specific probe further includes a universal sequence that can be recognized by the sequencing system;

   the 3'-end tail region of the specific probe comprises mismatched base;
   and/or, the 5-position hydroxyl group at the 5'-end nucleotide of the specific probe is substituted; preferably, the substituent group at the 5'-end of the specific probe is selected from a phosphate group or an adenosine group.

5. The enrichment method for a gene target region according to claim 1, wherein the ligation system in step (2) includes a single-stranded ligase, and the single-stranded ligase is preferably T4 RNA ligase or thermostable RNA ligase.

6. The enrichment method for a gene target region according to claim 1, wherein further comprises (3) Setting an enzyme-digestion site in a sequence that is not complementary to the target region on the specific probe, and adding an endonuclease after step (2) for excising a circular ligated product in the ligated product provided in step (2) at the enzyme-digestion site in order to provide a linear ligated product;
preferably, the enzyme-digestion site is uracil, and the endonuclease is ssDNA endonuclease or USER enzyme.

7. The enrichment method for a gene target region according to claim 6, wherein, further comprises (4) after the step (2), PCR amplifying the ligated product provided in the step (2);

   preferably, in the step (4), the PCR amplification primer has a sequence complementary to the specific probe but not complementary to the sequence of the target region,
   more preferably, the sequence complementary to the specific probe is a sequencing universal sequence; and/or,
   (5) after the step (3), PCR amplifying the ligated products provided in the steps (2) and (3);
   preferably, in the step (5), the PCR amplification primers have sequence complementary to the sequence on both sides of the enzyme-digestion site;
   more preferably, the sequences complementary to the sequences on both sides of the enzyme-digestion site are sequencing universal sequences;
   and/or, purification is carried out after any of the steps (1) to (5).

8. The enrichment method for a gene target region according to claim 7, wherein, further comprises (6) detecting the amplified product provided in step (4) or (5) using detection primer 1, detection primer 2 and probe 3, wherein at least one of the detection primer 1, detection primer 2 and probe 3 contains gene-specific sequences;

   preferably, the detection primer 1 comprises a gene-specific sequence, and the detection primer 2 and the probe 3 comprise a universal sequence;
   and/or, the detection primer 2 and/or probe 3 comprise gene-specific sequences, too;
   preferably, the probe 3 comprises a labelling molecule, and the sequence of the probe 3 is not complementary to that of the detection primer 1 or 2.

9. The enrichment method for a gene target region according to claim 1, wherein the enrichment method for a gene target region is used for nucleic acid detection.

10. A system for enriching target region of fragmented DNA, comprising specific probes and ligases suitable for the enrichment method for a gene target region according to any one of claims 1 to 9.

11. The system for enriching target region of fragmented DNA according to claim 10, wherein

   further comprises one or more of the following components: dNTPs coupled to labeled molecules, DNA polymerases, nucleases, and endonuclease;
   and/or, further comprises PCR amplification primers, the amplification primers have sequences that are at least partially complementary to the universal sequences of the specific probes;
and/or, further comprises detection primer 1, detection primer 2 and probe 3, at least one of which contains a gene-specific sequence.

FIG. 1

FIG. 2

## INTERNATIONAL SEARCH REPORT

| International application No. |
| --- |
| **PCT/CN2019/123892** |

**A.    CLASSIFICATION OF SUBJECT MATTER**

    C12Q 1/6806(2018.01)i

According to International Patent Classification (IPC) or to both national classification and IPC

**B.    FIELDS SEARCHED**

Minimum documentation searched (classification system followed by classification symbols)

    C12Q

Documentation searched other than minimum documentation to the extent that such documents are included in the fields searched

Electronic data base consulted during the international search (name of data base and, where practicable, search terms used)

    CNMED; TWABS; MOABS; TWMED; VEN; HKABS; CNABS; CPRSABS; SIPOABS; DWPI; VEN; CNTXT; EPTXT; USTXT; WOTXT; ISI-WEB OF SCIENCE; PUBMED; ELSEVIER; CNKI, WANFANG: 连接酶, 富集, 探针, 捕获, 延伸, 建库, 测序, 线性, 环状, 片段, cfDNA, extend+, ligase, NGS, sequencing, polymerase, probe, bp, library, capture, enrich

**C.    DOCUMENTS CONSIDERED TO BE RELEVANT**

| Category* | Citation of document, with indication, where appropriate, of the relevant passages | Relevant to claim No. |
| --- | --- | --- |
| X | CN 109593757 A (AMOY DIAGNOSTICS CO., LTD.) 09 April 2019 (2019-04-09) see abstract, claims 1-10, description paragraphs 58-76 | 1-12 |
| A | CN 108396057 A (CHONGQING CANCER INSTITUTE) 14 August 2018 (2018-08-14) entire document | 1-12 |
| A | CN 102373288 B (SHENG, Sitong et al.) 11 December 2013 (2013-12-11) entire document | 1-12 |
| A | CN 105780129 A (BGI TIANJIN CO., LTD. et al.) 20 July 2016 (2016-07-20) entire document | 1-12 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 June 2020** | **23 June 2020** |

| Name and mailing address of the ISA/CN | Authorized officer |
| --- | --- |
| **China National Intellectual Property Administration (ISA/ CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2019/123892** |

Box No. I   Nucleotide and/or amino acid sequence(s) (Continuation of item 1.c of the first sheet)

1.  With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
Information on patent family members

International application No.

**PCT/CN2019/123892**

| Patent document cited in search report | | | Publication date (day/month/year) | Patent family member(s) | | | Publication date (day/month/year) |
|---|---|---|---|---|---|---|---|
| CN | 109593757 | A | 09 April 2019 | WO | 2019062289 | A1 | 04 April 2019 |
| CN | 108396057 | A | 14 August 2018 | None | | | |
| CN | 102373288 | B | 11 December 2013 | CN | 102373288 | A | 14 March 2012 |
| CN | 105780129 | A | 20 July 2016 | CN | 105780129 | B | 11 June 2019 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019100024085 A